(19) Europäisches Patentamt
European Patent Office
Office européen des brevets

(11) Veröffentlichungsnummer: **0 392 351 A1**

(12) # EUROPÄISCHE PATENTANMELDUNG

(21) Anmeldenummer: **90106516.9**

(22) Anmeldetag: **05.04.90**

(51) Int. Cl.[5]: **C07C 317/18, C07C 317/36, C07C 317/40, C07D 279/16**

(30) Priorität: **12.04.89 DE 3911975**

(43) Veröffentlichungstag der Anmeldung: **17.10.90 Patentblatt 90/42**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Patsch, Manfred, Dr.**
**Fritz-Wendel-Strasse 4**
**D-6706 Wachenheim(DE)**
Erfinder: **Pandl, Klaus, Dr.**
**Schumannstrasse 18**
**D-6700 Ludwigshafen(DE)**
Erfinder: **Fischer, Martin, Dr.**
**Elbinger Weg 1**
**D-6700 Ludwigshafen(DE)**

(54) **Phenylsulfone und deren Cyclisierungsprodukte.**

(57) Phenylsulfone der Formel

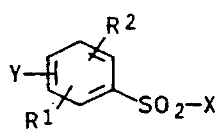

in der
$R^1$ Wasserstoff, Halogen, $C_1$-$C_4$-Alkyl, Nitro, Hydroxysulfonyl, Carboxyl, den Rest $-NR^3R^4$, in dem $R^3$ und $R^4$ unabhängig voneinander für Wasserstoff oder gegebenenfalls substituiertes $C_1$-$C_4$-Alkyl stehen, oder den Rest $S(O)_nR^5$, in dem n für 0 oder 2 und $R^5$ für gegebenenfalls substituiertes $C_1$-$C_4$-Alkyl, stehen,
$R^2$ Hydroxy, Mercapto oder den Rest $R^1$,
X den Rest

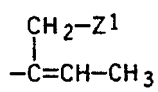 oder 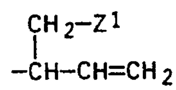

oder wenn der Rest $R^2$ orthoständig zur Gruppe $SO_2$-X steht, $R^2$ und X zusammen auch den Rest

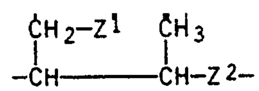 ,

wobei $Z^1$ für Hydroxy, Chlor, $C_1$-$C_4$-Alkanoloxy, $C_1$-$C_4$-Alkylsulfonyloxy, Phenylsulfonyloxy, o- oder p-Tolylsulfonyloxy oder Sulfato und $Z^2$ für Sauerstoff, Schwefel, Imino oder gegebenenfalls substituiertes $C_1$-$C_4$-Alkylimino, stehen und
Y Amino, $C_1$-$C_4$-Alkanoylamino oder Nitro
bedeuten, sowie ein Verfahren zu ihrer Herstellung.

EP 0 392 351 A1

Xerox Copy Centre

## Phenylsulfone und deren Cyclisierungsprodukte

Die vorliegende Erfindung betrifft neue Phenylsulfone der Formel I

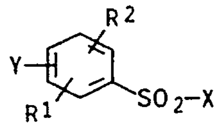

(I),

in der
$R^1$ Wasserstoff, Halogen, $C_1$-$C_4$-Alkyl, Nitro, Hydroxysulfonyl, Carboxyl, den Rest $-NR^3R^4$, in dem $R^3$ und $R^4$ gleich oder verschieden sind und unabhängig voneinander jeweils für Wasserstoff, $C_1$-$C_4$-Alkyl oder $C_2$-$C_4$-Alkyl, das durch Hydroxy, $C_1$-$C_4$-Alkanoyloxy, $C_1$-$C_4$-Alkoxy, Hydroxysulfonyl, Chlor oder Brom substituiert ist, stehen, oder den Rest $S(O)_nR^5$, in dem n für 0 oder 2 und $R^5$ für $C_1$-$C_4$-Alkyl oder $C_2$-$C_4$-Alkyl das durch Hydroxy, Chlor, Brom, $C_1$-$C_4$-Alkoxy, Hydroxysulfonyl oder Sulfato substituiert ist, stehen,
$R^2$ Hydroxy, Mercapto oder den Rest $R^1$,
X den Rest

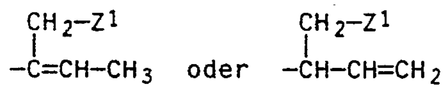

oder wenn der Rest $R^2$ orthoständig zur Gruppe $SO_2$-X steht, $R^2$ und X zusammen auch den Rest

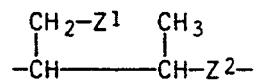

,

wobei $Z^1$ für Hydroxy, Chlor, $C_1$-$C_4$-Alkanoloxy, $C_1$-$C_4$-Alkylsulfonyloxy, Phenylsulfonyloxy, o- oder p-Tolylsulfonyloxy oder Sulfato und $Z^2$ für Sauerstoff, Schwefel, Imino oder $C_1$-$C_4$-Alkylimino, das gegebenenfalls durch Hydroxy, 2-Hydroxyethylthio oder 2-Hydroxyethylsulfonyl substituiert ist, stehen und
Y Amino, $C_1$-$C_4$-Alkanoylamino oder Nitro
bedeuten.

Aus der CH-A-492 766 sowie CH-A-492 767 sind Benzoylchloride bekannt, deren Substituenten in meta-Stellung chlorhaltige Butenylsulfonylreste, die gegebenenfalls noch eine Methylgruppe aufweisen, darstellen.

Aufgabe der vorliegenden Erfindung war es, neue Phenylsulfone bereitzustellen, die in einfacher Weise erhalten und vorteilhaft als Diazokomponente oder Reaktivanker verwendet werden können.

Demgemäß wurden die eingangs näher bezeichneten Phenylsulfone der Formel I gefunden.

$R^1$, $R^2$, $R^3$, $R^4$ und $R^5$ in Formel I bedeuten, z.B. Methyl, Ethyl, Propyl, Isopropyl, Butyl, Isobutyl oder sec-Butyl.

$R^3$, $R^4$ und $R^5$ bedeuten weiterhin z.B. 2-Hydroxyethyl, 2- oder 3-Hydroxypropyl, 2- oder 4-Hydroxybutyl, 2-Methoxyethyl, 2- oder 3-Methoxypropyl, 2- oder 4-Methoxybutyl, 2-Ethoxyethyl, 2- oder 3-Ethoxypropyl, 2- oder 4-Ethoxybutyl, 2-Propoxyethyl, 2- oder 3-Propoxypropyl, 2- oder 4-Propoxybutyl, 2-Isopropoxyethyl, 2- oder 3-Isopropoxypropyl, 2- oder 4-Isopropoxybutyl, 2-Butoxyethyl, 2- oder 3-Butoxypropyl, 2- oder 4-Butoxybutyl, 2-Hydroxysulfonylethyl, 2- oder 3-Hydroxysulfonylpropyl oder 2- oder 4-Hydroxysulfonylbutyl, 2-Chlorethyl, 2- oder 3-Chlorpropyl, 2- oder 4-Chlorbutyl, 2-Bromethyl, 2- oder 3-Brompropyl oder 2- oder 4-Brombutyl.

$R^5$ bedeutet weiterhin z.B. 2-Sulfatoethyl, 2- oder 3-Sulfatopropyl oder 2- oder 4-Sulfatobutyl.

$R^1$ bedeutet weiterhin z.B. Fluor, Chlor oder Brom.

Y bedeutet z.B. Formylamino, Acetylamino, Propionylamino, Butyrylamino oder Isobutyrylamino.

$R^1$ bedeutet, wie auch $Z^1$, weiterhin, z.B. Formyloxy, Acetyloxy, Propionyloxy, Butyryloxy oder Isobutyryloxy.

$Z^1$ bedeutet weiterhin z.B. Methylsulfonyloxy, Ethylsulfonyloxy, Propylsulfonyloxy, Isopropylsulfonyloxy oder Butylsulfonyloxy.

$Z^2$ bedeutet z.B. Methylimino, Ethylimino, Propylimino, Butylimino, 2-Hydroxyethylimino, 2-(2-Hydroxyethylthio)ethylimino oder 2-(2-Hydroxyethylsulfonyl)ethylimino.

Bevorzugt sind Phenylsulfone der Formel I, in der

$R^1$ Wasserstoff, Halogen oder den Rest $-NR^3R^4$, in dem $R^3$ und $R^4$ unabhängig voneinander für $C_2$-$C_4$-Alkyl, das durch Hydroxy substituiert ist, oder $R^3$ auch für Wasserstoff stehen, und

$R^2$ Hydroxy oder den Rest $R^1$ bedeuten.

Weiterhin bevorzugt sind Phenylsulfone der Formel I in der die Reste $R^2$ und X zusammen den Rest

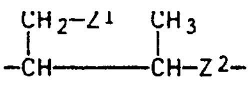 ,

bedeuten, wobei $Z^1$ die obengenannte Bedeutung besitzt und $Z^2$ für $C_1$-$C_4$-Alkylimino, das gegebenenfalls durch Hydroxy substituiert ist, steht.

Die erfindungsgemäßen Phenylsulfone der Formel I können vorteilhaft erhalten werden, indem man z.B. eine Sulfinsäure der Formel II

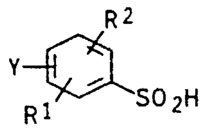 (II),

in der Y, $R^1$ und $R^2$ jeweils die obengenannte Bedeutung besitzen, mit Vinyloxiran der Formel III

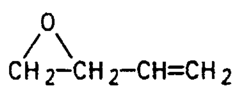 (III)

umsetzt.

Anstelle der Sulfinsäure II können auch deren Salze, insbesondere die Alkalisalze, z.B. das Natrium- oder Kaliumsalz, verwendet werden.

Die Umsetzung wird zweckmäßig so vorgenommen, daß man die Sulfinsäure der Formel III bei einem pH-Wert von ca. 5 bis 9 in Wasser löst und bei einer Temperatur von 20 bis 80 °C Vinyloxiran langsam zugibt. Das Molverhältnis von Sulfinsäure II : Vinyloxiran III beträgt dabei z.B. 1:1,1 bis 1:2.

Diejenigen Phenylsulfone der Formel I, in der, wenn der Rest $R^2$ orthoständig zur Gruppe $SO_2$-X steht, $R^2$ und X zusammen den Rest

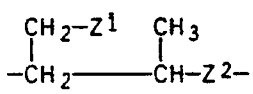

bedeuten, können z.B. durch Erwärmen von Verbindungen der Formel IV

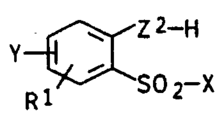 (IV),

in der $R^1$, X, Y und $Z^2$ jeweils die obengenannte Bedeutung besitzen, erhalten werden. Vorzugsweise arbeitet man dabei in einem inerten organischen Lösungsmittel, z.B. einem $C_1$-$C_8$-Alkohol, wie Methanol, Ethanol, Propanol, Isopropanol, Butanol, Isobutanol, Pentanol, Hexanol, Heptanol, Octanol oder 2-Ethylhexanol, einem Ether, wie Tetrahydrofuran oder Dioxan, oder einem Essigsäureester, wie Essigsäuremethyl- oder -ethylester, und hält eine Temperatur von 40 bis 120 °C ein.

Wenn der Rest Y in Formel I, II oder IV für Nitro oder $C_1$-$C_4$-Alkanoylamino steht, so kann er nach an sich bekannten Methoden, z.B. durch Reduktion der Nitrogruppe oder mittels Verseifung der Alkanoylaminogruppe, in die freie Aminogruppe übergeführt werden.

Zu denjenigen Verbindungen der Formel I, in der $Z^1$ für Chlor, $C_1$-$C_4$-Alkanoyloxy, $C_1$-$C_4$-Alkyl-

sulfonyloxy, Phenylsulfonyloxy, o- oder p-Tolylsulfonyloxy oder Sulfato steht, gelangt man beispielsweise, wenn man die entsprechenden Hydroxyverbindungen ($Z^1$ = Hydroxy) nach an sich bekannten Methoden mit Chlorierungsreagenzien (z.B. Thionylchlorid), $C_1$-$C_4$-Alkanoylhalogeniden, $C_1$-$C_4$-Alkylsulfonsäurehalogeniden, Phenylsulfonsäurehalogeniden, o- oder p-Tolylsulfonsäurehalogeniden oder Säureanhydriden umsetzt.

Bei den Sulfinsäuren der Formel II, den Verbindungen der Formel IV sowie bei Vinyloxiran der Formel III handelt es sich um an sich bekannte Verbindungen. Die Herstellung von Vinyloxiran ist z.B. in Ber. Dt. Chem. Ges. Band 66, Seite 335 bis 339, 1933, beschrieben.

Die neuen Phenylsulfone der Formel I sind wertvolle Zwischenprodukte für die Synthese von Farbstoffen. Sie eignen sich vorteilhaft als Reaktivanker oder auch als Diazokomponenten (mit Y = Amino).

Die folgenden Beispiele sollen die Erfindung näher erläutern.

Beispiel 1

Eine Lösung von 398 g p-Acetylaminobenzolsulfonsäure in 1000 ml Wasser wurde mit Natronlauge auf pH 7,5 gestellt. Bei 40 bis 45 °C tropfte man zu dieser Lösung 190 g Vinyloxiran innerhalb von 3 Stunden zu. Das Reaktionsgemisch wurde weitere 2 Stunden bei 40 bis 45 °C nachgerührt. Während der gesamten Reaktionszeit wurde der pH-Wert durch Zutropfen von 10 gew.%iger Schwefelsäure bei 7,5 gehalten. Nach dem Abkühlen wurde der Niederschlag abgesaugt und unter vermindertem Druck bei 50 °C getrocknet. Man isolierte 392 g der Verbindung der Formel

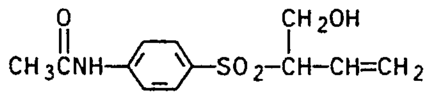

vom Fp.: 117 bis 119 °C.
H-NMR ($D_6$-DMSO): 2.1 (Intensität: 3), 3.7 (1), 3.9 (2), 5.0 (1; Triplett = OH), 10.4 (1), 5.1 (1), 5.3 (1), 5.7 (1), 8.8 (4).

Beispiel 2

63,8 g der Verbindung aus Beispiel 1 wurden in 250 g 10 gew.%iger Salzsäure 4 Stunden auf 100 °C erhitzt. Man ließ abkühlen, stellte den pH-Wert mit Natronlauge auf 9,5 bis 10 und extrahierte zweimal mit Essigester. Nach dem Einengen der organischen Phase verblieben 46 g eines Öls, das langsam kristallisierte und dann einen Schmelzpunkt von 75 bis 80 °C aufwies. Die resultierende Verbindung weist die Formel

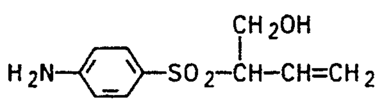

auf.

Beispiel 3

149,6 g m-Nitrobenzolsulfinsäure wurden bei pH 7,5 in 650 ml Wasser gelöst. Bei 40 bis 45 °C tropfte man 90 g Vinyloxiran zu und hielt den pH-Wert durch gleichzeitiges Zutropfen von 10 gew.%iger Schwefelsäure bei 7,5. Nach beendeter Umsetzung, was durch Dünnschichtchromatographie bestimmt werden kann, wurde zweimal mit 500 g Chloroform extrahiert. Durch Einengen der organischen Phase isolierte man 170 g eines Öls, das langsam kristallisierte. Aus n-Butanol umkristallisiert, schmolz das Produkt, das die Formel

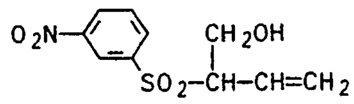

aufweist, bei 45 bis 48° C.
H-NMR ($D_6$-DMSO): 3.9 (2), 4.3 (1), 5.2 (1; Triplett = OH), 5.3 (1), 5.4 (1), 5.8 (1), 8.0 (1), 8.3 (1), 8.6 (2).

Beispiel 4

25,7 g der Verbindung aus Beispiel 3 wurden in 400 g Methanol und 2 g Propionsäure gelöst. Man gab 5 g Raney-Nickel zu und hydrierte bei 40° C unter 2 bar Überdruck. Nach beendeter Wasserstoff-Aufnahme wurde vom Katalysator abfiltriert und die Mutterlauge vom Lösungsmittel befreit. Es verblieben 20,2 g eines gelben Öls, das die Formel

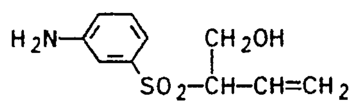

aufweist, und ohne weitere Reinigung zu Farbstoffsynthesen eingesetzt werden kann. Die Struktur wurde durch NMR-Spektren bestätigt.

Beispiel 5

In 400 ml Wasser wurden 85 g 2-Chlor-5-nitrobenzolsulfinsäure bei pH 7,5 gelöst. Bei 40 bis 45° C tropfte man 56 g Vinyloxiran innerhalb von 4 Stunden zu, wobei man durch gleichzeitige Zugabe von 10 gew.%iger Schwefelsäure den pH-Wert bei 7,5 hielt. Nach Abkühlung, Extraktion mit Essigester und Einengen isolierte man 45 g eines gelben Öls, das die Formel

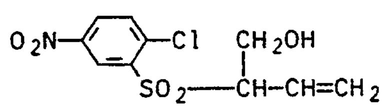

aufweist.
H-NMR ($D_6$-DMSO): 3.9 (2), 4.4 (1), 5.2 (1; Triplett = OH), 5.3 (1), 5.4 (1), 5.8 (1), 8.0 (1), 8.6 (1), 8.7 (1).
Cl ber.: 12,2 Cl gef.: 12,1 %

Beispiel 6

Eine Lösung von 50 g der Verbindung aus Beispiel 5 und 15 g Ethanolamin in 150 g Isopropanol wurden auf 60° C erhitzt. Nach 4 und 6 Stunden wurden jeweils 3 g Ethanolamin zugegeben. Das Lösungsmittel wurde unter vermindertem Druck abdestilliert. Es verblieben 68 g eines gelben Öls, das beim Erwärmen mit n-Butanol kristallisierte. Das isolierte und getrocknete Produkt hat einen Schmelzpunkt von 212 bis 216° C und entspricht nach NMR-Spektroskopie der Formel

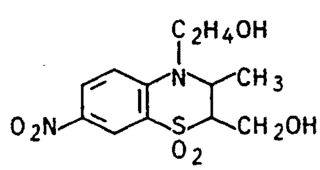

H-NMR ($D_6$-DMSO): 1.6 (3), 3.5 (2+1), 3.7 (2), 3.9 (2), 4.5 (1), 5.0 (1; Triplett = OH), 5.1 (1; Triplett = OH), 7.1 (1), 8.2 (1), 8.4 (1).

Beispiel 7

25 g der Verbindung aus Beispiel 6 wurden in 1000 g Methanol analog Beispiel 4 reduziert. Man isolierte 20 g eines Öls, das nach NMR-Spektroskopie folgende Struktur besitzt

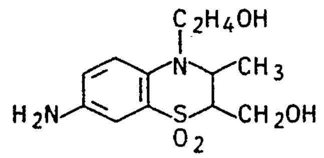

Beispiel 8

Eine Lösung von 58 g der Verbindung aus Beispiel 5 und 30 g N-Methylethanolamin in 150 g Isopropanol wurden 5 Stunden auf 65 bis 70° C erhitzt. Man goß auf Wasser und stellte den pH-Wert mit Salzsäure auf 1, wobei sich ein gelbes Öl abschied. Dann wurde mit Essigester extrahiert, die organische Phase über $Na_2SO_4$ getrocknet und unter vermindertem Druck eingeengt. Man isolierte 25 g eines gelben Öls, dem nach NMR-Spektroskopie die Formel

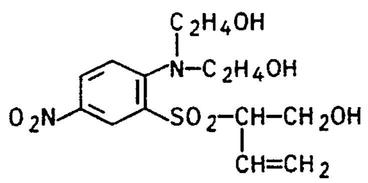

zuzuordnen ist.

**Ansprüche**

1. Phenylsulfone der Formel I

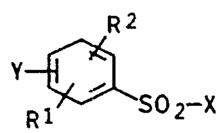

(I),

in der
$R^1$ Wasserstoff, Halogen, $C_1$-$C_4$-Alkyl, Nitro, Hydroxysulfonyl, Carboxyl, den Rest -$NR^3R^4$, in dem $R^3$ und $R^4$ gleich oder verschieden sind und unabhängig voneinander jeweils für Wasserstoff, $C_1$-$C_4$-Alkyl oder $C_2$-$C_4$-Alkyl, das durch Hydroxy, $C_1$-$C_4$-Alkanoyloxy, $C_1$-$C_4$-Alkoxy, Hydroxysulfonyl, Chlor oder Brom substituiert ist, stehen, oder den Rest $S(O)_nR^5$, in dem n für 0 oder 2 und $R^5$ für $C_1$-$C_4$-Alkyl oder $C_2$-$C_4$-Alkyl des durch Hydroxy, Chlor, Brom, $C_1$-$C_4$-Alkoxy, Hydroxysulfonyl oder Sulfato substituiert ist, stehen,
$R^2$ Hydroxy, Mercapto oder den Rest $R^1$,
X den Rest

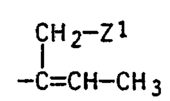

oder

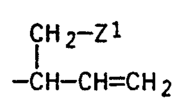

oder wenn der Rest $R^2$ orthoständig zur Gruppe $SO_2$-X steht, $R^2$ und X zusammen auch den Rest

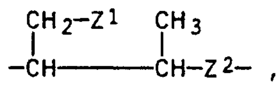

wobei $Z^1$ für Hydroxy, Chlor, $C_1$-$C_4$-Alkanoloxy, $C_1$-$C_4$-Alkylsulfonyloxy, Phenylsulfonyloxy, o- oder p-Tolylsulfonyloxy oder Sulfato und $Z^2$ für Sauerstoff, Schwefel, Imino oder $C_1$-$C_4$-Alkylimino, das gegebenenfalls durch Hydroxy, 2-Hydroxyethylthio oder 2-Hydroxyethylsulfonyl substituiert ist, stehen und
Y Amino, $C_1$-$C_4$-Alkanoylamino oder Nitro
bedeuten.

2. Phenylsulfone gemäß Anspruch 1, dadurch gekennzeichnet, daß
$R^1$ Wasserstoff, Halogen oder den Rest -$NR^3R^4$, in dem $R^3$ und $R^4$ unabhängig voneinander für $C_2$-$C_4$-Alkyl, das durch Hydroxy substituiert ist, oder $R^3$ auch für Wasserstoff stehen, und
$R^2$ Hydroxy oder den Rest $R^1$ bedueten.

3. Phenylsulfone gemäß Anspruch 1, dadurch gekennzeichnet, daß die Reste $R^2$ und X zusammen den Rest

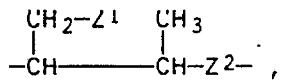

bedeuten, wobei $Z^1$ die in Anspruch 1 genannte Bedeutung besitzt und $Z^2$ für $C_1$-$C_4$-Alkylimino, das gegebenenfalls durch Hydroxy substituiert ist, steht.

4. Verfahren zur Herstellung der Phenylsulfone gemäß Anspruch 1, dadurch gekennzeichnet, daß man eine Sulfinsäure der Formel II

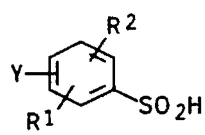

(II),

in der Y, $R^1$ und $R^2$ jeweils die in Anspruch 1 genannte Bedeutung besitzen, oder deren Salze mit Vinyloxiran der Formel III

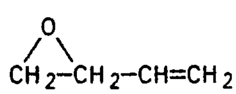

(III)

umsetzt.

Europäisches Patentamt

# EUROPÄISCHER RECHERCHENBERICHT

Nummer der Anmeldung

EP 90106516.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP - A1 - 0 007 880 (CHOAY S.A.) * Anspruch 1 * -- | 1,2 | C 07 C 317/18<br>C 07 C 317/36<br>C 07 C 317/40<br>C 07 D 279/16 |
| A | DE - B2 - 2 361 144 (RHONE-POULENC S.A.) * Anspruch 1 * -- | 1 | |
| A | EP - A1 - 0 101 933 (BAYER AG) * Zusammenfassung * -- | 1,3 | |
| D,A | CH - A - 492 766 (SANDOZ AG) * Patentanspruch * ---- | 1 | |

RECHERCHIERTE SACHGEBIETE (Int. Cl.5)

C 07 C 317/00
C 07 D 279/00

Der vorliegende Recherchenbericht wurde für alle Patentansprüche erstellt.

| Recherchenort | Abschlußdatum der Recherche | Prüfer |
|---|---|---|
| WIEN | 09-07-1990 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument